# EUROPEAN PATENT SPECIFICATION

(11) **EP 3 169 305 B1**
(45) Date of publication and mention of the grant of the patent: **07.04.2021**
(21) Application number: 15732754.5
(22) Date of filing: 06.07.2015
(51) Int. Cl.: A61K 8/49, A61Q 5/00, A61Q 5/06, A61Q 19/00

(54) **A USE OF ISOSORBIDE MONOOLEATE**
VERWENDUNG VON ISOSORBIDMONOOLEAT
UTILISATION DE MONOOLÉATE D'ISOSORBIDE

(30) Priority: 16.07.2014 EP 14177258
(43) Date of publication of application: 24.05.2017
(73) Proprietor: BASF SE, 67056 Ludwigshafen am Rhein (DE)
(72) Inventor: STOER, Claudia, 42699 Solingen (DE); NIEENDICK, Claus, 47807 Krefeld (DE); WEISSENEGGER, Markus, 40627 Düsseldorf (DE); PRINZ, Daniela, 41542 Dormagen (DE); WINZEK, Mirella, 52445 Titz (DE); SCHOSS, Jennifer, 40699 Erkrath (DE); DIERKER, Markus, 40593 Düsseldorf (DE); BOYXEN, Norbert, 47906 Kempen (DE); GRIESBACH, Ute, 40593 Düsseldorf (DE); SEIPEL, Werner, 40723 Hilden (DE); MAUER, Werner, 48324 Sendenhorst-Albersloh (DE)
(74) Representative: BASF IP Association
(86) International application number: PCT/EP2015/065326
(87) International publication number: WO 2016/008751

(56) References cited:
- EP-A1- 2 239 315
- WO-A1-2013/017255
- WO-A1-2013/017263
- WO-A1-2013/041388

## Description

The present invention relates to the use of a cosmetic composition comprising isosorbide monooleate for depositing isosorbide monooleate on human skin or hair according to claim 1. Cosmetic compositions, detergents and cleansers comprising isosorbide monooleate, i. e. the monoester of isosorbide and oleic acid, are known. Isosorbide monooleate has many advantageous properties which are the reason why isosorbide monooleate is used in cosmetic compositions, in detergents and in cleansers.

WO 2010/115565 **(**EP 2 239 315**)** discloses detergents and cleansers comprising isosorbide monoesters. In the example section a composition comprising 1 % by weight isosorbide monoester and 3 % by weight coco glucoside is disclosed.

WO 2013/041388 discloses cosmetic compositions comprising isosorbide monooleate, specifically disclosed is a composition comprising 1 % by weight isosorbide monooleate and 1.5 % by weight coco glucoside (Figure 4). This composition has good foaming performance.

**European patent application no.** 14154978 filed on February 13, 2014 (BASF internal file no. PF 75368) discloses a microemulsion comprising isosorbide monooleate and caprylyl/capryl glucoside and lauryl glucoside. It is disclosed that isosorbide monooleate which is used commercially is generally a mixture of monoester, diester, isosorbide and fatty acids, mainly oleic acid.

The treatment of human skin or hair with a cosmetic composition, e. g. a shampoo or a shower gel or a body wash composition, can lead to a depletion of the skin or hair of protecting lipids. This can lead to dry skin, to brittle hair, and to other unwanted effects.

The problem underlying the present invention is to avoid or reduce these negative effects.

This problem is solved by the use of a cosmetic composition comprising isosorbide monooleate for depositing isosorbide monooleate on human skin or hair according to claim 1. This use is a subject of the present invention.

One embodiment of the present invention is the use according to the present invention, wherein the cosmetic composition comprises isosorbide monooleate in an amount of 0.2 to 3 % by weight, preferably 0.4 to 2.5 % by weight, more preferably 0.5 to 2 % by weight.

One embodiment of the present invention is the use according to the present invention (i. e. according to the use which is the subject of the present invention or according to any of the embodiments described in the previous paragraphs), wherein the cosmetic composition comprises a surfactant which is different from isosorbide monooleate selected from the group consisting of an anionic surfactant, a cationic surfactant, a nonionic surfactant an amphoteric or zwitterionic surfactant and mixtures thereof, preferably in an amount of 2 to 40 % by weight, more preferably 5 to 30 % by weight, more preferably 10 to 20 % by weight.

One embodiment of the present invention is the use according to the embodiment described in the previous paragraph, wherein the surfactant which is different from isosorbide monooleate is an anionic surfactant and wherein the cosmetic composition further comprises a cationic polymer suitable for use in a cosmetic composition, preferably in an amount of 0.1 to 1 % by weight, more preferably 0.1 to 0.8 % by weight, more preferably 0.15 to 0,5 % by weight, and wherein the cationic polymer is preferably selected from the group consisting of a polyquaternium (according to INCI nomenclature) and a cationic guar derivative, more preferably selected from the group consisting of polyquaternium 7, polyquaternium 10 and a cationic guar derivative.

One embodiment of the present invention is the use according to the present invention (i. e. according to the use which is the subject of the present invention or according to any of the embodiments described in the previous paragraphs), wherein the cosmetic composition further comprises one or more cosmetically acceptable ingredients.

One embodiment of the present invention is the use according to the present invention (i. e. according to the use which is the subject of the present invention or according to any of the embodiments described in the previous paragraphs), wherein the cosmetic composition is selected from the group consisting of a shampoo, a shower gel, a body-wash composition and a skin or hair conditioner.

One embodiment of the present invention is the use according to the present invention (i. e. according to the use which is the subject of the present invention or according to any of the embodiments described in the previous paragraphs), wherein this use results in a lipid layer enhancing effect on the skin or hair or in a moisturizing effect on the skin or hair or in a humectant effect on the skin or hair or in a protecting effect on the skin or hair or in a caring effect on the skin or hair.

One embodiment of the present invention is the use according to the present invention (i. e. according to the use which is the subject of the present invention or according to any of the embodiments described in the previous paragraphs), wherein the isosorbide monooleate is a mixture comprising pure isosorbide monooleate in an amount of 65 - 95 % by weight, preferably 65 - 85 % by weight, more preferably 65 - 75 % by weight.

One embodiment of the present invention is the use according to the embodiments described in the previous paragraph, wherein the fatty acid moieties in the pure isosorbide monooleate comprise more than 65 % by weight, preferably more than 70 % by weight, more preferably more than 75 % by weight, oleic acid moieties.

One embodiment of the present invention is the use according to the present invention (i. e. according to the use which is the subject of the present invention or according to any of the embodiments described in the previous paragraphs), wherein the use is realized in a process comprising contacting human skin or hair with a cosmetic composition comprising isosorbide monooleate and rinsing off the cosmetic composition comprising isosorbide monooleate with water, so that, after rinsing off the cosmetic composition, isosorbide monooleate is left deposited on the human skin or hair, in an amount of 0.01 to 10 µg/cm², more preferably 0.1 to 5 µg/cm², more preferably 0.5 to 1 µg/cm².

Isosorbide monooleate can be made as disclosed in WO 2010/115565 or by other known esterification methods.

Cosmetic compositions comprising isosorbide monooleate can be made as disclosed in WO 2013/041388 or by other known methods for making cosmetic compositions.

Isosorbide monooleate according to the present invention generally is a mixture comprising (pure) isosorbide monooleate as major component and additionally isosorbide dioleate, oleic acid and isosorbide. In this mixture the oleic acid, which is generally obtained from plants, also is a mixture comprising oleic acid as major component and other fatty acids in minor amounts. The term pure isosorbide monooleate means the monoester of isosorbide with a fatty acid mixture comprising oleic acid as major component. Here, major component means more than 65 % by weight, preferably more than 70 % by weight, more preferably more than 75 % by weight, oleic acid. Therefore, in one embodiment of the present invention the isosorbide monooleate is a mixture comprising pure isosorbide monooleate in an amount of 65 - 95 % by weight, preferably 65 - 85 % by weight, more preferably 65 - 75 % by weight. The amounts in % by weight can be determined by gas chromatography (GC) with appropriate calibration. In this pure isosorbide monooleate the fatty acid moieties comprise more than 65 % by weight, preferably more than 70 % by weight, more preferably more than 75 % by weight, oleic acid moieties. The amounts in % by weight can be determined by gas chromatography (GC) with appropriate calibration.

**Cosmetic compositions** are to be understood here as meaning all compositions which are exclusively or primarily intended to be used externally on the human body or in its oral cavity for cleaning, care, protection, maintaining a good condition, perfuming, changing the appearance or for the purposes of influencing body odor.

The cosmetic compositions according to the invention can be in particular formulations for bodycare, e.g. a body milk, creams, lotions, sprayable emulsions, products for eliminating body odor etc. They can be surfactant-containing formulations such as e.g. foam and shower baths, hair shampoos and care rinses. Depending on the intended application, the cosmetic formulations can comprise a series of further auxiliaries and additives, for example surfactants, oil bodies, emulsifiers, pearlescent waxes, consistency regulators, thickeners, superfatting agents, stabilizers, polymers, fats, waxes, lecithins, phospholipids, biogenic active ingredients, UV light protection factors, antioxidants, deodorants, antiperspirants, antidandruff agents, film formers, swelling agents, insect repellents, self-tanning agents, tyrosine inhibitors (depigmentation agents), hydrotropes, solubilizers, preservatives, perfume oils, dyes etc., which are listed below by way of example.

**Surfactants** Surface-active substances which may be present are anionic, nonionic, cationic and/or amphoteric or zwitterionic surfactants. In surfactant-containing cosmetic preparations, for example shower gels, foam baths, shampoos etc., preferably at least one anionic surfactant is present. The fraction of surfactants here is usually about 1 to 30, preferably 5 to 25 and in particular 10 to 20% by weight.

Typical examples of **anionic surfactants** are soaps, alkylbenzenesulfonates, alkanesulfonates, olefinsulfonates, alkyl ether sulfonates, glycerol ether sulfonates, α-methyl ester sulfonates, sulfo fatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids, such as, for example, acyl lactylates, acyl tartrates, acyl glutamates and acyl aspartates, alkyl oligoglycoside sulfates, protein fatty acid condensates (in particular wheat-based vegetable products) and alkyl (ether) phosphates. If the anionic surfactants contain polyglycol ether chains, these can have a conventional homolog distribution, but preferably have a narrowed homolog distribution. Typical examples of **nonionic surfactants** are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partially oxidized alk(en)yl oligoglycosides and glucuronic acid derivatives, fatty acid N-alkylglucamides, protein hydrolyzates (in particular wheat-based vegetable products), polyol fatty acid esters, sugar esters, sorbitan esters, polysorbates and amine oxides. If the nonionic surfactants contain polyglycol ether chains, these can have a conventional homolog distribution, but preferably have a narrowed homolog distribution. Typical examples of **cationic surfactants** are quaternary ammonium compounds, such as, for example, dimethyldistearylammonium chloride, and ester quats, in particular quaternized fatty acid trialkanolamine ester salts. Typical examples of **amphoteric or zwitterionic surfactants** are alkylbetaines, alkylamidobetaines, aminopropionates, aminoglycinates, imidazoliniumbetaines and sulfobetaines. Said surfactants are exclusively known compounds. As regards structure and preparation of these substances, reference may be made to relevant review works in this field. Typical examples of particularly suitable mild, i.e. particularly skin-compatible, surfacatants are fatty alcohol polyglycol ether sulfates, monoglyceride sulfates, mono- and/or dialkyl sulfosuccinates, fatty acid isethionates, fatty acid sarcosinates, fatty acid taurides, fatty acid glutamates, α-olefinsulfonates, ether carboxylic acids, alkyl oligoglucosides, fatty acid glucamides, alkylamidobetaines, amphoacetals and/or protein fatty acid condensates, the latter preferably based on wheat proteins.

**Oil bodies:** Bodycare compositions, such as creams, lotions and milks, usually comprise oil bodies and emollients. Suitable oil bodies and emollients are disclosed in WO 2013/041388.

**Fats and waxes:** Fats and waxes are added to bodycare products as care substances and also in order to increase the consistency of the cosmetics. Suitable fats and waxes are disclosed in WO 2013/041388.

Suitable **thickeners** are disclosed in WO 2013/041388.

**UV light protection factors** are to be understood as meaning, for example, organic substances (light protection filters) that are present in liquid or crystalline form at room temperature and which are able to absorb ultraviolet rays and release the absorbed energy again in the form of longer-wave radiation, e.g. heat. Suitable UV light protection factors are disclosed in WO 2013/041388.

**Biogenic active ingredients** are to be understood as meaning, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, such as e.g. prune extract, bambara nut extract and vitamin complexes.

**Deodorizing active ingredients** counteract, mask or eliminate body odors. Suitable deodorizing active ingredients are disclosed in WO 2013/041388.

Suitable insect repellents, self-tanning agents, tyrosine inhibitors, preservatives, perfumes, pearlescent waxes, superfatting agents, stabilizers, hydrotropes, which can be present in the composition according to the present invention are disclosed in WO 2013/041388.

### Examples

For measuring the deposition of isosorbide monooleate on a substrate a formulation containing isosorbide monooleate was applied to this substrate. Then, the treated area was rinsed with a defined amount of water and extracted with an organic solvent in order to determine the amount of isosorbide monooleate that remained on the surface after the rinse cycle. The extract was analyzed by gas chromatography (GC) and the amount of isosorbide monooleate deposited on the surface was calculated. The measurements were carried out as described in the experimental section of WO 2013/007473.

As substrate the human skin (forearm) of probands (in-vivo deposition) as well as a skin substitute (collagen layer) (in-vitro deposition) was used. The rinse-cycle (dilution with water) imitated the process of having a shower.

The following formulations were used for the deposition measurements (in-vitro as well as in-vivo). The isosorbide monooleate had a content of about 77 % by weight monoester in which the acid moiety had a content of more than 75 % by weight oleic acid moieties. The example containing 2% by weight of isosorbide monooleate is a reference example.

### Composition of Formulations Tested

| **Components (INCl)** | **Amount (% by weight)** | **Amount (% by weight)** | **Amount (% by weight)** | **Amount (% by weight)** |
|---|---|---|---|---|
| Sodium Laureth Sulfate | 11.2 | 11.2 | 11.2 | 11.2 |
| Sodium Chloride | 1.0-1.8 | 1.0-1.8 | 1.0-1.8 | 1.0-1.8 |
| Cocoamidopropyl Betaine | 1.5 | 1.5 | 1.5 | 1.5 |
| Natriumbenzoat | 0.5 | 0.5 | 0.5 | 0.5 |
| Polyquaternium-7 | 0.2 | 0.2 | 0.2 | 0.2 |
| Coco Glucoside | 1.5 | 1.5 | 1.5 | 1.5 |
| Isosorbide Monooleate | 0.5 | 1 | 1.5 | 2 |
| Citric acid | 0.2 | 0.2 | 0.2 | 0.2 |
| water | Up to 100 | Up to 100 | Up to 100 | Up to 100 |

### In-Vitro Deposition: Results

A concentration dependency was observed: The higher the amount of isosorbide monooleate in the formulation the higher the deposition rate of this substance on the skin / skin substitute.

| Concentration of isosorbide monooleate in the formulation (% by weight) | In-vitro deposition of Isosorbide monooleate on the skin substitute per area (µg/cm²) |
|---|---|
| 0.5 | 3.7 |
| 1 | 7.3 |
| 1.5 | 8.6 |
| 2 | 15.8 |

### In-Vivo Deposition: Results

The formulation with 1 % by weight isosorbide monooleate was tested. It could be shown that isosorbide monooleate remained in small amounts on the skin after rinse-off.

| Concentration of isosorbide monooleate in the formulation (% by weight) | In-vivo Deposition of isosorbide monooleate on the skin per area (µg/cm²) |
|---|---|
| 1 | 0.7 |

## Claims

1. The use of a cosmetic composition comprising isosorbide monooleate for depositing isosorbide monooleate on human skin or hair,
wherein the use is realized in a process comprising
contacting human skin or hair with a cosmetic composition comprising isosorbide monooleate and
rinsing off the cosmetic composition comprising isosorbide monooleate with water, so that, after rinsing off the cosmetic composition, isosorbide monooleate is left deposited on the human skin or hair in an amount of 0.01 to 10 µg/cm².

2. The use according to claim 1, wherein the cosmetic composition comprises isosorbide monooleate in an amount of 0.2 to 3 % by weight, preferably 0.4 to 2.5 % by weight, more preferably 0.5 to 2 % by weight.

3. The use according to claim 1 or 2, wherein the cosmetic composition comprises a surfactant which is different from isosorbide monooleate selected from the group consisting of an anionic surfactant, a cationic surfactant, a nonionic surfactant an amphoteric or zwitterionic surfactant and mixtures thereof, preferably in an amount of 2 to 40 % by weight, more preferably 5 to 30 % by weight, more preferably 10 to 20 % by weight.

4. The use according to claim 3, wherein the surfactant which is different from isosorbide monooleate is an anionic surfactant and wherein the cosmetic composition further comprises a cationic polymer suitable for use in a cosmetic composition, preferably in an amount of 0.1 to 1 % by weight, more preferably 0.1 to 0.8 % by weight, more preferably 0.15 to 0,5 % by weight, and wherein the cationic polymer is preferably selected from the group consisting of a polyquaternium and a cationic guar derivative, more preferably selected from the group consisting of polyquaternium 7, polyquaternium 10 and a cationic guar derivative.

5. The use according to any of claims 1 to 4, wherein the cosmetic composition further comprises one or more cosmetically acceptable ingredients.

6. The use according to any of claims 1 to 5, wherein the cosmetic composition is selected from the group consisting of a shampoo, a shower gel, a body-wash composition and a skin or hair conditioner.

7. The use according to any of claims 1 to 6, wherein this use results in a lipid layer enhancing effect on the skin or hair or in a moisturizing effect on the skin or hair or in a humectant effect on the skin or hair or in a protecting effect on the skin or hair or in a caring effect on the skin or hair.

8. The use according to any of claims 1 to 7, wherein the isosorbide monooleate is a mixture comprising pure isosorbide monooleate in an amount of 65 - 95 % by weight, preferably 65 - 85 % by weight, more preferably 65 - 75 % by weight.

9. The use according to claim 8, wherein the fatty acid moieties in the pure isosorbide monooleate comprise more than 65 % by weight, preferably more than 70 % by weight, more preferably more than 75 % by weight, oleic acid moieties.

10. The use according to any of claims 1 to 9, wherein the isosorbide monooleate is left deposited in an amount of 0.1 to 5 µg/cm², preferably 0.5 to 1 µg/cm².

## Patentansprüche

1. Verwendung einer kosmetischen Zusammensetzung, die Isosorbidmonooleat umfasst, zur Abscheidung von Isosorbidmonooleat auf menschlicher Haut oder menschlichem Haar,
wobei die Verwendung in einem Verfahren realisiert wird, bei dem man
menschliche Haut oder menschliches Haar mit einer kosmetischen Zusammensetzung, die Isosorbidmonooleat umfasst, in Kontakt bringt und
die kosmetische Zusammensetzung, die Isosorbidmonooleat umfasst, mit Wasser abspült, so dass nach dem Abspülen der kosmetischen Zusammensetzung Isosorbidmonooleat auf der menschlichen Haut bzw.
dem menschlichen Haar in einer Menge von 0,01 bis 10 µg/cm² abgeschieden bleibt.

2. Verwendung nach Anspruch 1, wobei die kosmetische Zusammensetzung Isosorbidmonooleat in einer Menge von 0,2 bis 3 Gew.-%, vorzugsweise 0,4 bis 2,5 Gew.-%, weiter bevorzugt 0,5 bis 2 Gew.-%, umfasst.

3. Verwendung nach Anspruch 1 oder 2, wobei die kosmetische Zusammensetzung ein von Isosorbidmonooleat verschiedenes Tensid aus der Gruppe bestehend aus einem anionischen Tensid, einem kationischen Tensid, einem nichtionischen Tensid, einem amphoteren oder zwitterionischen Tensid und Mischungen davon umfasst, vorzugsweise in einer Menge von 2 bis 40 Gew.-%, weiter bevorzugt 5 bis 30 Gew.-%, weiter bevorzugt 10 bis 20 Gew.-%.

4. Verwendung nach Anspruch 3, wobei es sich bei dem von Isosorbidmonooleat verschiedenen Tensid um ein anionisches Tensid handelt und wobei die kosmetische Zusammensetzung ferner ein zur Verwendung in einer kosmetischen Zusammensetzung geeignetes kationisches Tensid umfasst, vorzugsweise in einer Menge von 0,1 bis 1 Gew.-%, weiter bevorzugt 0,1 bis 0,8 Gew.-%, weiter bevorzugt 0,15 bis 0,5 Gew.-%, und wobei das kationische Polymer vorzugsweise aus der Gruppe bestehend aus einem Polyquaternium und einem kationischen Guarderivat, weiter bevorzugt aus der Gruppe bestehend aus Polyquaternium 7, Polyquaternium 10 und einem kationischen Guarderivat, ausgewählt ist.

5. Verwendung nach einem der Ansprüche 1 bis 4, wobei die kosmetische Zusammensetzung ferner einen oder mehrere kosmetisch unbedenkliche Inhaltsstoffe umfasst.

6. Verwendung nach einem der Ansprüche 1 bis 5, wobei die kosmetische Zusammensetzung aus der Gruppe bestehend aus einem Shampoo, einem Duschbad, einer Körperwaschzusammensetzung und einem Haut- oder Haarkonditionierungsmittel ausgewählt ist.

7. Verwendung nach einem der Ansprüche 1 bis 6, wobei diese Verwendung zu einer lipidschichtverstärkenden Wirkung auf die Haut oder das Haar oder zu einer befeuchtenden Wirkung auf die Haut oder das Haar oder zu einer feuchthaltenden Wirkung auf die Haut oder das Haar oder zu einer schützenden Wirkung auf die Haut oder das Haar oder zu einer pflegenden Wirkung auf die Haut oder das Haar führt.

8. Verwendung nach einem der Ansprüche 1 bis 7, wobei es sich bei dem Isosorbidmonooleat um ein Gemisch handelt, das reines Isosorbidmonooleat in einer Menge von 65-95 Gew.-%, vorzugsweise 65-85 Gew.-%, weiter bevorzugt 65-75 Gew.-%, umfasst.

9. Verwendung nach Anspruch 8, wobei die Fettsäuregruppierungen in dem reinen Isosorbidmonooleat mehr als 65 Gew.-%, vorzugsweise mehr als 70 Gew.-%, weiter bevorzugt mehr als 75 Gew.-%, Ölsäuregruppierungen umfassen.

10. Verwendung nach einem der Ansprüche 1 bis 9, wobei das Isosorbidmonooleat in einer Menge von 0,1 bis 5 µg/cm², vorzugsweise 0,5 bis 1 µg/cm², abgeschieden bleibt.

## Revendications

1. Utilisation d'une composition cosmétique comprenant du monooléate d'isosorbide pour le dépôt de monooléate d'isosorbide sur la peau humaine ou les cheveux humains,
l'utilisation étant réalisée dans un procédé comprenant
la mise en contact de peau humaine ou de cheveux humains avec une composition cosmétique comprenant du monooléate d'isosorbide et
le rinçage de la composition cosmétique comprenant du monooléate d'isosorbide avec de l'eau, de sorte qu'après le rinçage de la composition cosmétique, du monooléate d'isosorbide est laissé déposé sur la peau humaine ou les cheveux humains en une quantité de 0,01 à 10 µg/cm².

2. Utilisation selon la revendication 1, la composition cosmétique comprenant du monooléate d'isosorbide en une quantité de 0,2 à 3 % en poids, préférablement 0,4 à 2,5 % en poids, plus préférablement 0,5 à 2 % en poids.

3. Utilisation selon la revendication 1 ou 2, la composition cosmétique comprenant un tensioactif qui est différent du monooléate d'isosorbide choisi dans le groupe constitué par un tensioactif anionique, un tensioactif cationique, un tensioactif non ionique, un tensioactif amphotère ou zwitterionique et des mélanges correspondants, préférablement en une quantité de 2 à 40 % en poids, plus préférablement 5 à 30 % en poids, plus préférablement 10 à 20 % en poids.

4. Utilisation selon la revendication 3, le tensioactif qui est différent du monooléate d'isosorbide étant un tensioactif anionique et la composition cosmétique comprenant en outre un polymère cationique approprié pour une utilisation dans une composition cosmétique, préférablement en une quantité de 0,1 à 1 % en poids, plus préférablement 0,1 à 0,8 % en poids, plus préférablement 0,15 à 0,5 % en poids, et le polymère cationique étant préférablement choisi dans le groupe constitué par un polyquaternium et un dérivé de guar cationique, plus préférablement choisi dans le groupe constitué par le polyquaternium 7, le polyquaternium 10 et un dérivé de guar cationique.

5. Utilisation selon l'une quelconque des revendications 1 à 4, la composition cosmétique comprenant en outre un ou plusieurs ingrédients acceptables sur le plan cosmétique.

6. Utilisation selon l'une quelconque des revendications 1 à 5, la composition cosmétique étant choisie dans le groupe constitué par un shampooing, un gel douche, une composition de lavage corporel et un revitalisant pour la peau ou les cheveux.

7. Utilisation selon l'une quelconque des revendications 1 à 6, cette utilisation résultant en un effet d'augmentation de la couche lipidique sur la peau ou les cheveux ou en un effet hydratant sur la peau ou les cheveux ou en un effet humectant sur la peau ou les cheveux ou en un effet protecteur sur la peau ou les cheveux ou en un effet de soin sur la peau ou les cheveux.

8. Utilisation selon l'une quelconque des revendications 1 à 7, le monooléate d'isosorbide étant un mélange comprenant du monooléate d'isosorbide pur en une quantité de 65 à 95 % en poids, préférablement 65 à 85 % en poids, plus préférablement 65 à 75 % en poids.

9. Utilisation selon la revendication 8, les groupements de type acide gras dans le monooléate d'isosorbide pur comprenant plus de 65 % en poids, préférablement plus de 70 % en poids, plus préférablement plus de 75 % en poids, de groupements de type acide oléique.

10. Utilisation selon l'une quelconque des revendications 1 à 9, le monooléate d'isosorbide étant laissé déposé en une quantité de 0,1 à 5 µg/cm², préférablement 0,5 à 1 µg/cm².
